## Europäisches Patentamt

(19) **European Patent Office**

**Office européen des brevets**

(11) Numéro de publication : **0 269 598 B1**

---

(12) **FASCICULE DE BREVET EUROPEEN**

(45) Date de publication du fascicule du brevet :
**10.04.91 Bulletin 91/15**

(51) Int. Cl.$^5$ : **A61K 9/50,** A61K 31/74,
A61K 9/16, A61K 31/785

(21) Numéro de dépôt : **87870141.6**

(22) Date de dépôt : **08.10.87**

---

(54) **Agents thérapeutiques contre la leishmaniose.**

---

(30) Priorité : **08.10.86 FR 8613999**

(43) Date de publication de la demande :
**01.06.88 Bulletin 88/22**

(45) Mention de la délivrance du brevet :
**10.04.91 Bulletin 91/15**

(84) Etats contractants désignés :
**AT BE CH DE FR GB IT LI LU NL SE**

(56) Documents cités :
EP-A- 0 007 895
EP-A- 0 064 967
CHEMICAL ABSTRACTS, vol. 98, no. 21, 23 mai 1983, page 364, résumé no. 185458v, Columbus, Ohio, US; J. KREUTER:
"Physicochemical characterization of polyacrylic nanoparticles", & INT. J. PHARM. 1983, 14(1), 43-58
CHEMICAL ABSTRACTS, vol. 104, no. 20, 19 mai 1986, page 384, résumé no. 174575x, Columbus, Ohio, US; M.S. EL-SAMALIGY et al.: "Polyalkyl cyanoacrylate nanocapsules", & J. PHARM. PHARMACOL. 1986, 38(3), 216-18

(56) Documents cités :
CHEMICAL ABSTRACTS, vol. 105, no. 21, 24 novembre 1986, page 404, résumé no. 187450y, Columbus, Ohio, US; L. GOLIGHTLY et al.: "The effect of macromolecular conjugates of daunorubicin on nuclear ultrastructure in Trypanosoma brucei rhodesiense", & CELL BIOL. INT. REP. 1986, 10(9), 717-25
CHEMICAL ABSTRACTS, vol. 72, no. 5, 2 février 1970, page 174, résumé no. 20279n, Columbus, Ohio, US; L.M. GORDEEVA et al.: "Effect of Flagyl, dehydroemetine, and other chemotherapeutic drugs individually and in combination against Balantidium coli in culture", & MED. PARAZITOL. PARAZIT. BOLEZ. 1969, 38(3), 278-81
CHEMICAL ABSTRACTS, vol. 73, 1970, page 226, résumé no. 129274d, Columbus, Ohio, US; P.J. VAN DIJCK et al.: "Effect of polyacrylic acid on experimental malaria and trypanosomiasis in mice", & ANN. TROP. MED. PARASITOL. 1970, 64(1), 5-9

(73) Titulaire : **N.V. SOPAR S.A.**
**Rue Ducale, 29**
**B-1000 Bruxelles (BE)**

(72) Inventeur : **Osuna Carillo de Albornoz, Antonio**
**Santa Barbara 18**
**E-18001 Granada (ES)**
Inventeur : **Castanys, Santiago**
**Antigua Carretera de Malaga 18**
**E-18015 Granada (ES)**

(74) Mandataire : **Grisar, Daniel et al**
**c/o Office Kirkpatrick 4, square de Meeûs**
**B-1040 Bruxelles (BE)**

---

EP 0 269 598 B1

## Description

La présente invention a pour objet de nouveaux agents thérapeutiques contre la leishmaniose, et des compositions pharmaceutiques les comprenant.

On désigne par le terme de leishmaniose un groupe d'affections qui sont provoquées chez l'homme et les autres mammifères par différentes espèces de parasites du genre Leishmania. Les espèces les plus importantes sont Leishmania tropica, Leishmania aethiopica, Leishmania mexicana, Leishmania braziliensis et Leishmania donovani (F. Wunderlich et E. Schurr, Biologie in unserer Zeit 14 (1984), 111-120).

Les agents pharmaceutiques connus et universellement administrés contre cette affection, qui sont notamment la déhydroémétine et l'amphotéricine B, exercent, en dehors de l'activité souhaitée, des effets secondaires très toxiques et indésirables.

L'invention a dès lors pour but de procurer de nouveaux agents thérapeutiques contre la leishmaniose, qui soient moins toxiques à activité égale que les principes actifs classiques ou qui aient une plus haute activité pour une toxicité à peu près égale.

Par les documents EP-B-0 007 895 et EP-B-0 064 967, on connaît des particules submicroscopiques obtenues par la polymérisation micellaire de cyanoacrylates d'alkyle, et contenant sous forme absorbée ou adsorbée une substance biologiquement active.

Comme il est dit dans ces documents, ces particules polymériques submicroscopiques qui contiennent une substance biologiquement active, peuvent être utilisées pour le traitement de nombreuses maladies et notamment de certains types de cancer. Ces particules contenant une substance biologiquement active peuvent être administrées par voie parentérale et offrent notamment comme avantage d'être biodégradables et d'exercer une action thérapeutique prolongée et plus efficace que celle qui peut être obtenue par l'administration de la substance biologiquement active seule (non contenue dans des particules). De plus, la toxicité des particules contenant la substance est moindre que celle de la substance biologiquement active seule.

Il a été découvert avec surprise que les particules submicroscopiques préparées par la polymérisation de dérivés d'acide cyanoacrylique, suivant les procédés décrits dans les documents EP-B-0 007 895 et EP-B-0 064 967, sont actives contre les formes connues de la leishmaniose et cela même lorsque ces particules ne contiennent aucun agent pharmaceutique connu pour son activité contre la leishmaniose. Il a été constaté qu'administrées par voie parentérale, ces particules exemptes d'autre substance exercent contre la leishmaniose un effet thérapeutique très prononcé et semblable à celui des médicaments connus comme actifs contre cette maladie tout en n'ayant pas la toxicité de ces médicaments.

Du fait qu'il est connu à la suite de nombreuses recherches que ces particules submicroscopiques à base de polymères de cyanoacrylates d'alkyle ne sont que peu ou pas toxiques, du moins pour ce qui concerne les quantités administrées à des fins thérapeutiques, ces particules submicroscopiques pures exemptes d'autres principes actifs conviennent comme agents thérapeutiques efficaces contre la leishmaniose.

Il a été constaté, en outre, que l'administration conjointe de certains agents chimiothérapeutiques connus pour leur activité contre la leishmaniose et de particules submicroscopiques à base de polymères de cyanoacrylate d'alkyle permet d'atteindre un effet thérapeutique supérieur à celui qui est obtenu avec les dites particules submicroscopiques seules ou avec les dits agents chimiothérapeutiques seuls.

Lorsque les dits agents chimiothérapeutiques sont utilisés conjointement avec les dites particules submicroscopiques, la toxicité et les effets secondaires indésirables des agents chimiothérapeutiques sont nettement réduits lorsque ces derniers se trouvent sous forme adsorbée ou absorbée dans les particules submicroscopiques.

L'invention a donc pour objet un agent thérapeutique pour utilisation dans le traitement de la leishmaniose qui consiste en des particules submicroscopiques dont le diamètre est inférieur à 500 nanomètres, susceptibles d'être obtenues par la polymérisation micellaire d'au moins un cyanoacrylate d'alkyle.

De manière plus spécifique, il est fait usage de particules submicroscopiques à base de cyanoacrylate d'alkyle polymère dont la chaîne alkyle, linéaire ou ramifiée, compte 1 à 12 atomes de carbone et en particulier 4 à 7 atomes de carbone.

Selon une forme d'exécution, les particules submicroscopiques sont susceptibles d'être obtenues par la copolymérisation d'au moins deux cyanoacrylates d'alkyle différents.

De manière particulière, ces particules peuvent être obtenues par la polymérisation micellaire de cyanoacrylate d'isobutyle (cyanoacrylate de méthyl-2-propyle) et/ou de cyanoacrylate d'isohexyle (cyanoacrylate d'éthyl-2-butyle).

Les particules submicroscopiques qui constituent les agents thérapeutiques suivant l'invention peuvent être préparées suivant les procédés décrits dans les documents EP-B-0 007 895 ou EP-B-0 064 967 et consistent en les polymères mentionnés formés par polymérisation ou copolymérisation.

Il est surprenant que ces particules submicroscopiques polymériques pures exemptes d'autres principes

2

actifs exercent une action thérapeutique très efficace contre la leishmaniose, cette action étant semblable à celle des médicaments connus comme actifs contre cette maladie.

Cette action thérapeutique peut éventuellement être rendue encore plus efficace lorsqu'il est fait usage de particules submicroscopiques polymériques contenant au moins une substance connue comme étant active contre la leishmaniose.

En particulier, on peut alors faire usage de particules chargées de déhydroémétine et/ou d'amphotéricine B.

Les agents thérapeutiques conformes à l'invention contiennent les particules submicroscopiques contenant ou non une autre substance antiparasitaire, sous forme de suspension ou de solution colloïdale dans des solutions physiologiquement compatibles ou bien sont des poudres sèches qui sont mises en suspension ou en solution colloïdale avant l'administration dans des solutions physiologiquement compatibles, par exemple une solution de chlorure de sodium ou de dextrose.

L'invention a également pour objet des compositions pharmaceutiques pour utilisation dans le traitement de la leishmaniose comprenant au moins un agent thérapeutique suivant l'invention en association avec un véhicule consistant en une solution physiologiquement compatible.

L'invention a également pour objet de telles compositions pharmaceutiques comprenant, en outre, de l'amphotéricine B.

EXEMPLE 1.-

L'activité d'agents thérapeutiques selon l'invention a été éprouvée sur des rats blancs (poids moyen : 340 g) auxquels a été inoculée une dose unique de $50 \times 10^6$ agents causaux de l'affection (Leishmania donovani).

Les animaux d'épreuve ont été traités au moyen de divers agents thérapeutiques 30 jours après l'inoculation.

Chaque traitement a consisté en trois injections intracardiaques, chacune de 1 ml, administrées à intervalles de 7 jours, sauf pour la déhydroémétine seule, qui, en raison de sa forte toxicité cardiaque, a été injectée par voie intrapéritonéale.

Doses expérimentales injectées (ayant chacune une teneur appropriée en principe actif) :

a) déhydroémétine en solution aqueuse à 1,2 mg par ml,

b) particules submicroscopiques à base de cyanoacrylate d'isohexyle polymérisé ne contenant pas d'autre substance active, en suspension aqueuse à 24 mg par ml,

c) particules submicroscopiques à base de cyanoacrylate d'hexyle polymérisé contenant 5% de déhydroémétine (chaque ml de la suspension contient 24 mg de particules submicroscopiques et 1,2 mg de déhydroémétine).

Après le traitement, les agents causaux présents dans la rate ont été déterminés et comparés à ceux retrouvés chez des animaux inoculés de même, mais non traités (témoins).

| Résultats | | |
|---|---|---|
| Traitement | Agents causaux de l'affection, par gramme de rate | Réduction des agents causaux (%) |
| – | $36,015 \times 10^6$ | – |
| a | $4,870 \times 10^6$ | 86,5 |
| b | $6,550 \times 10^6$ | 81,8 |
| c | $0,585 \times 10^6$ | 98,4 |

## EXEMPLE 2.-

D'autres rats, d'un poids moyen de 300 g, ont reçu une dose unique de 12 × 10⁶ agents causaux de l'affection.

Les agents thérapeutiques ont été injectés par voie intraveineuse ; le traitement a débuté 30 jours après l'inoculation et a consisté en 3 injections de 0,5 ml, administrées à intervalles de 7 jours.

Doses expérimentales injectées :

a') déhydroémétine en solution aqueuse, à raison de 1,75 mg par kg de rat,

b') particules submicroscopiques à base de cyanoacrylate d'isohexyle polymérisé, en suspension aqueuse, à raison de 35 mg par kg de rat,

c') particules submicroscopiques à base de cyanoacrylate d'isohexyle polymérisé contenant de la déhydroémétine (à raison de 1,75 mg de déhydroémétine et 35 mg de particules submicroscopiques par kg de rat).

Après traitement, les agents causaux présents par 100 cellules nucléées normales de rate ont été déterminés et comparés à ceux retrouvés chez des témoins non traités.

| Résultats | | |
|---|---|---|
| Traitement | Agents causaux de l'affection, par 100 cellules de rate | Réduction des agents causaux (%) |
| - | 28 | - |
| a' | 16 | 42,86 |
| b' | 12 | 57,1 |
| c' | 12 | 57,1 |

## EXEMPLE 3.-

L'activité d'agents thérapeutiques selon l'invention a été testée d'une manière similaire à celle de l'exemple 2, mais de l'amphotéricine B a été employée au lieu de déhydroémétine en tant que substance biologiquement active.

Des rats d'un poids moyen de 300 g ont reçu une dose unique de 12 × 10⁶ agents causaux de l'affection.

Les agents thérapeutiques ont été injectés par voie intraveineuse ; le traitement a débuté 30 jours après l'inoculation et a consisté en 3 injections de 1 ml, administrée à intervalles de 7 jours.

Doses expérimentales administrées :

a") amphotéricine B en solution aqueuse, à raison de 2,5 mg par kg de rat,

b") particules submicroscopiques à base de cyanoacrylate d'isohexyle polymérisé, en suspension aqueuse, à raison de 35 mg par kg de rat,

c") particules submicroscopiques à base de cyanoacrylate d'isohexyle polymérisé contenant de l'amphotéricine B (à raison de 2,5 mg d'amphotéricine B et 35 mg de particules submicroscopiques par kg de rat.

Après traitement, les agents causaux présents par gramme de rate ont été déterminés et comparés à ceux retrouvés chez des témoins.

| Résultats | | |
|---|---|---|
| Traitement | Agents causaux de l'affection, par gramme de rate | Réduction des agents causaux (%) |
| – | $11 \times 10^6$ | – |
| a" | $2,8 \times 10^6$ | 74,5 |
| b" | $4,8 \times 10^6$ | 56,5 |
| c" | $2 \times 10^6$ | 81,8 |

## Revendications

1. Agent thérapeutique pour utilisation dans le traitement de la leishmaniose, caractérisé en ce qu'il consiste en des particules submicroscopiques dont le diamètre est inférieur à 500 nanomètres, susceptibles d'être obtenues par la polymérisation micellaire d'au moins un cyanoacrylate d'alkyle dont la chaîne alkyle, linéaire ou ramifiée, contient de 1 à 12 atomes de carbone.

2. Agent thérapeutique suivant la revendication 1, caractérisé en ce qu'il consiste en des particules submicroscopiques susceptibles d'être obtenues par la polymérisation d'au moins un cyanoacrylate d'alkyle dont la chaîne alkyle contient de 4 à 7 atomes de carbone.

3. Agent thérapeutique suivant l'une quelconque des revendications 1 et 2, caractérisé en ce qu'il consiste en des particules submicroscopiques susceptibles d'être obtenues par la copolymérisation d'au moins deux cyanoacrylates d'alkyle différents.

4. Agent thérapeutique suivant la revendication 2, caractérisé en ce qu'il consiste en des particules submicroscopiques susceptibles d'être obtenues par la polymérisation de cyanoacrylate de méthyl-2-propyle.

5. Agent thérapeutique suivant la revendication 2, caractérisé en ce qu'il consiste en des particules submicroscopiques susceptibles d'être obtenues par la polymérisation de cyanoacrylate d'éthyl-2-butyle.

6. Composition pharmaceutique pour utilisation dans le traitement de la leishmaniose, caractérisée en ce qu'elle comprend au moins un agent thérapeutique suivant l'une quelconque des revendications précédentes en association avec un véhicule consistant en une solution physiologiquement compatible.

7. Composition pharmaceutique suivant la revendication 6, caractérisée en ce qu'elle comprend, en outre, de l'amphotéricine B.

## Ansprüche

1. Heilmittel für die Verwendung in der Behandlung von Leishmaniose, dadurch gekennzeichnet, daß es submikroskopische Teilchen mit einem Durchmesser kleiner als 500 nm enthält, die durch micellare Polymerisation von mindestens einem Alkylcyanoacrylat, dessen lineare oder verzweigte Alkylkette 1 bis 12 Kohlenstoffatome enthält, ethalten werden können.

2. Heilmittel nach Anspruch 1, dadurch gekennzeichnet, daß es submikroskopische Teilchen enthält, die durch Polymerisation von mindestens einem Alkylcyanoacrylat, dessen Alkylkette 4 bis 7 Kohlenstoffatome enthält, erhalten werden können

3. Heilmittel nach einem der Ansprüche 1 und 2, dadurch gekennzeichnet, daß es submikroskopische Teilchen enthält, die durch Copolymerisation von mindestens zwei verschiedenen Alkylcyanoacrylaten erhalten werden können.

4. Heilmittel nach Anspruch 2, dadurch gekennzeichnet, daß es submikroskopische Teilchen enthält, die durch Polymerisation von 2-Methyl-propyl-cyanoacrylat erhalten werden können.

5

5. Heilmittel nach Anspruch 2, dadurch gekennzeichnet, daß es submikroskopische Teilchen enthält, die durch Polymerisation von 2-Ethyl-butyl-cyanoacrylat erhalten werden können.

6. Pharmazeutische Zusammensetzung für die Verwendung in der Behandlung von Leishmaniose, dadurch gekennzeichnet, daß sie mindestens ein Heilmittel nach einem der vorhergehenden Ansprüche zusammen mit einem geeigneten Träger in einer physiologisch verträglichen Lösung umfaßt.

7. Pharmazeutische Zusammensetzung nach Anspruch 6, dadurch gekennzeichnet, daß sie außerdem Amphotericin B enthält.

## Claims

1. A therapeutic agent for use in the treatment of leishmaniosis characterised in that it consists of submicroscopic particles which are of a diameter of less than 500 nanometres, which are obtainable by the micellar polymerisation of at least one alkyl cyanoacrylate in which the straight or branched alkyl chain contains from 1 to 12 carbon atoms.

2. A therapeutic agent according to claim 1 characterised in that it consists of submicroscopic particles obtainable by the polymerisation of at least one alkyl cyanoacrylate in which the alkyl chain contains from 4 to 7 carbon atoms.

3. A therapeutic agent according to either one of claims 1 and 2 characterised in that it consists of submicroscopic particles obtainable by the copolymerisation of at least two different alkyl cyanoacrylates.

4. A therapeutic agent according to claim 2 characterised in that it consists of submicroscopic particles obtainable by the polymerisation of 2-methyl propyl cyanoacrylate.

5. A therapeutic agent according to claim 2 characterised in that it consists of submicroscopic particles obtainable by the polymerisation of 2-ethyl butyl cyanoacrylate.

6. A pharmaceutical composition for use in the treatment of leishmaniosis characterised in that it comprises at least one therapeutic agent according to any one of the preceding claims in association with a carrier consisting of a physiological compatible solution.

7. A pharmaceutical composition according to claim 6 characterised in that it further comprises amphotericin B.